# EUROPÄISCHE PATENTSCHRIFT

(11) **EP 3 585 930 B1**
(45) Veröffentlichungstag und Bekanntmachung des Hinweises auf die Patenterteilung: **03.08.2022**
(21) Anmeldenummer: 18705863.1
(22) Anmeldetag: 09.02.2018
(51) Int. Cl.: A47L 15/42, A47L 15/00, D06F 34/22, G01N 33/18, G01N 21/49

(54) **WASSERFÜHRENDES HAUSHALTSGERÄT UND VERFAHREN ZUM BETREIBEN EINES WASSERFÜHRENDEN HAUSHALTSGERÄTS**
WATER-CONDUCTING DOMESTIC APPLIANCE AND METHOD FOR OPERATING A WATER-CONDUCTING DOMESTIC APPLIANCE
APPAREIL MÉNAGER À CIRCULATION D'EAU ET PROCÉDÉ POUR FAIRE FONCTIONNER UN APPAREIL MÉNAGER À CIRCULATION D'EAU

(30) Priorität: 21.02.2017 DE 102017202777
(43) Veröffentlichungstag der Anmeldung: 01.01.2020
(73) Patentinhaber: BSH Hausgeräte GmbH, 81739 München (DE)
(72) Erfinder: HERRMANN, Ingo, 71292 Friolzheim (DE); HÜBEL, Alexander, 71229 Leonberg (DE)
(86) Internationale Anmeldenummer: PCT/EP2018/053301
(87) Internationale Veröffentlichungsnummer: WO 2018/153696

(56) Entgegenhaltungen:
- EP-A1- 1 199 973
- EP-A1- 2 857 572
- WO-A1-2004/027414
- DE-A1-102004 015 387
- US-A- 5 374 834
- US-A1- 2012 011 663
- US-A1- 2015 293 056

## Beschreibung

Die vorliegende Erfindung betrifft ein wasserführendes Haushaltsgerät und ein Verfahren zum Betreiben eines wasserführenden Haushaltsgeräts.

Herkömmliche wasserführende Haushaltsgeräte, wie eine Geschirrspülmaschine oder eine Waschmaschine, verwenden über die Hausanschlussleitung zugeführtes Leitungswasser. Die Wasserhärte des Leitungswassers hängt von verschiedenen Faktoren, wie der Wasseraufbereitung sowie den geologischen Gegebenheiten, ab. Die Wasserhärte des Leitungswassers kann zeitlichen Schwankungen unterliegen und sich sogar innerhalb eines Tages verändern. EP 2 857 572 A1 offenbart eine elektrochemisch arbeitende Enthärtungseinrichtung, die auch für wasserführende Haushaltsgeräte verwendbar ist.

Die Wasserhärte kann einen negativen Einfluss auf wasserführende Haushaltsgeräte haben. Sie kann beispielsweise dazu führen, dass Waschmittel und/oder Spülmittel eine reduzierte Wirksamkeit aufweisen. Für ein zufriedenstellendes Ergebnis eines Waschvorgangs und/oder eines Spülvorgangs muss die Menge des Waschmittels und/oder des Spülmittels gemäß der Wasserhärte angepasst werden. Da jedoch häufig nur sehr grobe und veraltete Werte für die Wasserhärte des Leitungswassers vorliegen, muss in diesen Fällen von dem höchsten Wert innerhalb eines qualitativen Wertebereichs (weich, mittel, hart) ausgegangen werden, was zu einer Verschwendung von Waschmittel und/oder Spülmittel führen kann. Dies kann sich sowohl ökonomisch als auch ökologisch negativ auswirken. Die Wasserhärte kann auch ein Verkalken von Haushaltsgeräten bewirken, was die Lebensdauer der Haushaltsgeräte stark reduzieren kann. Neben der Wasserhärte kann auch der pH-Wert des Leitungswassers eine wichtige Rolle für derartige Haushaltsgeräte spielen.

Die Wasserhärte ist eine Funktion der Konzentration von in dem Wasser gelösten Ionen, insbesondere den sogenannten "Härtebildnern", wie Calcium und Magnesium. Eine Möglichkeit, die Wasserhärte zu bestimmen, ist daher die Bestimmung der jeweiligen lonenkonzentration.

Zur Bestimmung einer lonenkonzentration in einer Flüssigkeit sind verschiedene Methoden bekannt. Komplexe Methoden erlauben eine quantitative und ionenselektive Messung. Beispielsweise gelingt dies mit ionenselektiven Elektroden und Optoden. Hier sind jedoch spezielle Funktionalisierungsschichten erforderlich, die nur eine begrenzte Lebensdauer aufweisen und daher nicht für den Dauereinsatz geeignet sind. Auch mit Voltammetrie lassen sich quantitative und ionenselektive Messungen durchführen, wobei hier aufgrund von elektrochemischen Prozessen eine Alterung der Elektroden den Dauereinsatz beeinträchtigt. Impedanzspektroskopie erfordert gut kontrollierte Bedingungen, da beispielsweise leitfähige Partikel die Messergebnisse sehr stark beeinflussen, weshalb die Methode nur für den Laboreinsatz geeignet ist. Elektrophorese hingegen benötigt lange Messzeiten und hat einen hohen Wartungsaufwand. Diese Methoden sind daher für den Einsatz in wasserführenden Haushaltsgeräten weniger geeignet.

US 2015/293056 A1 offenbart eine Vorrichtung, die zum Erfassen von in einer Flüssigkeit gelösten geladenen Ziel-Partikeln eingerichtet ist. Dabei wird ein elektrisches Feld verwendet, so dass die geladenen Ziel-Partikel sich in der Flüssigkeit bewegen, wodurch ein Konzentrationsgradient der geladenen Ziel-Partikel in der Flüssigkeit erzeugt wird. Eine Sensitivität der Erfassung der geladenen Ziel-Partikel lässt sich hierdurch erhöhen. US 5 374 834 A offenbart ein ladungsgekoppeltes Element, das auf Basis einer Separierung von Ionen in einer Flüssigkeitsprobe eine Bestimmung der geladenen Bestandteile der Flüssigkeitsprobe ermöglicht.

Vor diesem Hintergrund besteht eine Aufgabe der vorliegenden Erfindung darin, ein verbessertes wasserführendes Haushaltsgerät zu schaffen.

Demgemäß wird ein wasserführendes Haushaltsgerät, insbesondere Geschirrspülmaschine, mit einer Sensorvorrichtung zum Bestimmen eines lonenprofils einer Flüssigkeit vorgeschlagen. Die Sensorvorrichtung weist einen Rohrleitungsabschnitt zum Durchströmen mit der Flüssigkeit, eine erste Elektrode, eine zweite Elektrode und eine Anzahl von Sensorelementen auf. Die erste Elektrode, die zweite Elektrode und der Rohrleitungsabschnitt sind derart relativ zueinander angeordnet, dass sich durch Anlegen einer Spannung zwischen der ersten Elektrode und der zweiten Elektrode ein elektrisches Feld in zumindest einem Teilstück des Rohrleitungsabschnitts ausbildet. Die Sensorelemente sind an einem Rand des Rohrleitungsabschnitts angeordnet und zum Ermitteln einer lokalen Flüssigkeitseigenschaft zum Bestimmen des lonenprofils der durch den Rohrleitungsabschnitt geleiteten Flüssigkeit eingerichtet.

Ein solches wasserführendes Haushaltsgerät weist den Vorteil auf, dass das lonenprofil des dem Haushaltsgerät zugeführten Wassers jederzeit schnell und exakt bestimmbar ist. Somit kann auf veränderte Bedingungen sofort reagiert werden. Das lonenprofil umfasst dabei beispielsweise nicht nur Informationen über eine Gesamtionenkonzentration, sondern kann auch Informationen zu der Konzentration von unterschiedlichen lonensorten umfassen. Mit dem Begriff lonensorte sind hierbei einerseits unterschiedliche Elemente als auch unterschiedliche Ladungszahlen bezeichnet. Die Sensorvorrichtung ermöglicht damit eine selektive und quantitative Bestimmung von lonenkonzentrationen. Beispielsweise umfasst das lonenprofil die Informationen, dass in dem Wasser eine Konzentration von 1 mmol/l Ca²⁺-Ionen, eine Konzentration von 0,2 mmol/l Mg²⁺-Ionen und 0,05 mmol/l anderer Kationen vorhanden ist.

Ein weiterer Vorteil der Sensorvorrichtung ist, dass diese sehr robust und langlebig ist und sich daher unter anderem für den Dauereinsatz eignet. Beispielsweise kann der Rohrleitungsabschnitt eine Rohrleitung aus Kunststoff umfassen. Die erste und die zweite Elektrode sind beispielsweise durch zwei Metallplatten ausgebildet. Wird eine elektrische Spannung zwischen den Metallplatten angelegt, so bildet sich zwischen diesen ein elektrisches Feld aus. Die Metallplatten bilden somit einen Plattenkondensator. Beispielsweise ist die Rohrleitung zwischen den beiden Metallplatten angeordnet. Sofern die Rohrleitung keinen Faraday'schen Käfig bildet, durchdringt das elektrische Feld dann auch den Innenraum der Rohrleitung. Das Teilstück der Rohrleitung, welches von dem elektrischen Feld durchdrungen wird, kann auch als Messstrecke bezeichnet werden. Wenn die Flüssigkeit durch die Rohrleitung geleitet wird, beeinflusst das elektrische Feld insbesondere die Verteilung von mobilen geladenen Teilchen in der Flüssigkeit, wie beispielsweise Ionen. Da die Elektroden dabei selbst nicht in Kontakt mit der Flüssigkeit stehen, finden an diesen keine elektrochemischen Prozesse statt. Eine Alterung der Sensorvorrichtung aufgrund derartiger Effekte ist somit ausgeschlossen.

Die Sensorelemente zum Bestimmen einer lokalen Eigenschaft der Flüssigkeit sind bevorzugt im Bereich der Messstrecke an dem Rohrleitungsabschnitt angeordnet. In Abhängigkeit davon, welcher Art die Sensorelemente sind, sind diese in Kontakt mit dem Fluid in dem Innenraum der Rohrleitung, also beispielsweise mit der hindurchgeleiteten Flüssigkeit. Die Sensorelemente umfassen beispielsweise optische und/oder elektrische Sensoren. Die lokale Flüssigkeitseigenschaft ist insbesondere zumindest mit der lokalen lonenkonzentration korreliert. Beispielsweise beeinflusst die lonenkonzentration die Leitfähigkeit und den Brechungsindex der Flüssigkeit. So kann beispielsweise von einem lokal unterschiedlichen Brechungsindex auf lokal unterschiedliche lonenkonzentrationen geschlossen werden. Auch Leitfähigkeitsmessungen sind hierfür geeignet.

Für ein binäres System, wie Wasser mit darin gelöstem Kochsalz, ist beispielsweise der Brechungsindex linear mit der Salzkonzentration korreliert, solange die Salzkonzentration nicht zu groß ist. Unter der Annahme, dass nur genau diese eine Ionensorte vorhanden ist, lässt sich daher nach einer Eichung direkt auf den Salzgehalt schließen. Unter einer Eichung wird vorliegend der Zusammenhang zwischen der lonenkonzentration und der Flüssigkeitseigenschaft, wie zum Beispiel dem Brechungsindex, verstanden.

Für die Bestimmung des lonenprofils aus einer Anzahl von solchen Messwerten kommen beispielweise mathematische Modelle zum Einsatz. Die Messwerte an unterschiedlichen Positionen der Messtrecke unterscheiden sich dabei aufgrund des Effekts, den das elektrische Feld auf die Flüssigkeit, insbesondere die darin gelösten geladenen Teilchen, hat.

In einer ruhenden Flüssigkeit führt ein elektrisches Feld dazu, dass sich Ionen in der Flüssigkeit entlang den elektrischen Feldlinien zum jeweils entgegengesetzten Pol hin bewegen, d.h. Anionen in Richtung zum Plus-Pol und Kationen in Richtung zum Minus-Pol. Die Geschwindigkeit, mit der die Ionen migrieren, hängt dabei vor allem von der Ladung, der Stärke des elektrischen Feldes und der Größe des Ions ab. Nach einer gewissen Zeit bildet sich eine Gleichgewichtsverteilung der Ionen in der Flüssigkeit aus. Das Gleichgewicht wird durch die Stärke der elektrostatischen Anziehungskraft und der in die entgegengesetzte Richtung wirkende Diffusion aufgrund der Konzentrationsunterschiede bestimmt. Dabei verhält sich jede Ionensorte unterschiedlich. Dies betrifft sowohl die Gleichgewichtsverteilung als auch den zeitlichen Verlauf, bis diese erreicht ist. Insbesondere aus der Messung des zeitlichen Verlaufs lässt sich daher auf die Ionensorte rückschließen.

Es ist zu berücksichtigen, dass die lokale Flüssigkeitseigenschaft zunächst lediglich eine Gesamtinformation vermittelt, also beispielsweise eine Gesamtionenkonzentration. Erst durch die Kombination von mehreren Messungen, beispielsweise an unterschiedlichen Positionen entlang der Messstrecke, lässt sich das lonenprofil bestimmen. Aus mathematischer Sicht werden insbesondere so viele unabhängige Messwerte ermittelt, wie Variablen bestimmt werden sollen. Da das lonenprofil sich nicht eindeutig aus kollektiven Eigenschaften, wie dem Brechungsindex oder der Leitfähigkeit, bestimmen lässt, sondern nur über Modellrechnungen zugänglich ist, ist es vorteilhaft, eine möglichst große Zahl an unabhängigen Messungen zu verwenden. Die wird beispielsweise erreicht, indem eine große Zahl von Sensorelementen an unterschiedlichen Positionen der Messstrecke angeordnet wird. Ferner lassen sich unterschiedliche Ergebnisse auch durch Messungen bei unterschiedlicher elektrischer Feldstärke erzielen.

Durch die Messung der Flüssigkeitseigenschaft an mehreren Positionen der Messtrecke entlang der Strömungsrichtung der Flüssigkeit lässt sich der zeitliche Verlauf bestimmen, da die Flüssigkeit eine in Abhängigkeit der Strömungsgeschwindigkeit bekannte Zeit benötigt, um von einer Einlassöffnung der Messtrecke bis zu einem jeweiligen Sensorelement zu strömen.

Vorzugsweise sind die Dimensionen der Rohrleitung im Bereich der Messstrecke so bemessen, dass sich in dem Teilstück eine laminare Strömung ausbildet.

Vorliegend ist es zur Bestimmung eines lonenprofils vorteilhaft, dass vorliegend keine funktionalisierten Schichten benötigt werden, deren Lebensdauer begrenzt ist. Weiterhin ist vorteilhaft, dass beispielsweise Metallpartikel auf das Ergebnis keinen Einfluss haben. Ferner finden keine elektrochemischen Prozesse statt, die eine Lebensdauer von mit der Flüssigkeit in Kontakt stehenden Bauteilen beeinträchtigen können. Auch wird eine Zusammensetzung der Flüssigkeit nicht verändert und es wird kein Verbrauchsmaterial benötigt. Der Betrieb der Sensorvorrichtung erfolgt damit ohne laufende Kosten. Auch kann die Flüssigkeit nach dem Durchströmen der Sensorvorrichtung weiterverwendet werden.

Die Sensorelemente zum Bestimmen der lokalen Flüssigkeitseigenschaft sind an einem Rand des Rohrleitungsabschnitts angeordnet.

Beispielsweise sind die Sensorelemente in das Rohr, das die Messstrecke bildet, eingelassen. Um die Strömung der Flüssigkeit dabei nicht zu beeinflussen, ist es vorteilhaft, wenn das Sensorelement dabei bündig mit einer Innenseite des Rohrs abschließt.

Gemäß einer weiteren Ausführungsform des wasserführenden Haushaltsgeräts ist die lokale Flüssigkeitseigenschaft eine lokale elektrische Leitfähigkeit und die Sensorelemente sind zum Bestimmen der lokalen elektrischen Leitfähigkeit eingerichtet.

Die elektrische Leitfähigkeit einer Flüssigkeit ist insbesondere dazu geeignet, auf in der Flüssigkeit gelöste mobile Ladungsträger, wie beispielsweise Ionen, rückzuschließen.

Gemäß einer weiteren Ausführungsform des wasserführenden Haushaltsgeräts ist die lokale Flüssigkeitseigenschaft ein lokaler Brechungsindex und die Sensorelemente sind zum Bestimmen des lokalen Brechungsindex eingerichtet.

Gemäß einer weiteren Ausführungsform des wasserführenden Haushaltsgeräts sind die Sensorelemente zum Bestimmen des lokalen Brechungsindex als integrierte optische Strukturen ausgebildet.

Diese Ausführungsform hat insbesondere den Vorteil, dass die Sensorelemente klein und robust sind. Beispielsweise sind die Sensorelemente als Ringresonatoren und/oder als Interferometer auf der Basis von Silizium-Photonik ausgebildet.

Alternativ können die Sensorelemente auch als plasmonische Strukturen ausgebildet sein. Diese sind insbesondere für Veränderungen, die sehr nahe an der Grenzfläche der Struktur zu dem benachbarten Medium liegen, sehr empfindlich.

Gemäß einer weiteren Ausführungsform des wasserführenden Haushaltsgeräts sind die Sensorelemente entlang einer Strömungsrichtung der Flüssigkeit in dem Rohrleitungsabschnitt angeordnet.

Gemäß einer weiteren Ausführungsform des wasserführenden Haushaltsgeräts ist die zweite Elektrode als Kathode ausgebildet, wobei die Sensorelemente entlang einer Strömungsrichtung der Flüssigkeit in dem Rohrleitungsabschnitt in einer Reihe auf der Seite der Kathode angeordnet sind.

Mit dieser Ausführungsform lässt sich ein zeitlicher Verlauf der lonenkonzentration in Abhängigkeit einer Wirkdauer des elektrischen Felds auf die Flüssigkeit auf der Seite der Kathode bestimmen.

Gemäß einer weiteren Ausführungsform des wasserführenden Haushaltsgeräts sind die Sensorelemente in einer Ebene parallel zu dem elektrischen Feld zwischen der ersten Elektrode und der zweiten Elektrode seitlich in dem Rohrleitungsabschnitt in einer Sensormatrix angeordnet.

Mit dieser Ausführungsform lässt sich ein vollständiges Abbild der lokalen Flüssigkeitseigenschaft in der Strömungsrichtung und in Richtung des elektrischen Feldes ermitteln. Diese Ausführungsform weist daher eine besonders hohe Genauigkeit der Bestimmung des lonenprofils der Flüssigkeit auf.

Gemäß einer weiteren Ausführungsform des wasserführenden Haushaltsgeräts ist eine Berechnungseinheit zum Berechnen des lonenprofils der Flüssigkeit in Abhängigkeit der ermittelten lokalen Flüssigkeitseigenschaft und dem elektrischen Feld vorgesehen.

Die Berechnungseinheit verwendet zur Berechnung insbesondere mathematische Modelle, die beispielsweise durch empirische Versuchsreihen ermittelt wurden und durch analytische und/oder algebraische Funktionen darstellbar sind. Ferner kann die Berechnungseinheit dazu eingerichtet sein, durch iterative Optimierungsmethoden ein Ergebnis zu ermitteln. Dabei wird beispielsweise eine Zielgröße, wie die mittlere quadratische Abweichung RMSE (von engl. *root mean square error*) der theoretischen Kurve von den Messwerten minimiert, indem die Parameterwerte des Modells variiert werden.

Die Berechnungseinheit kann hardwaretechnisch und/oder auch softwaretechnisch implementiert sein. Bei einer hardwaretechnischen Implementierung kann die Berechnungseinheit als eine Überprüfungsvorrichtung oder als Teil des Backends, zum Beispiel als Computer oder als Mikroprozessor ausgebildet sein. Bei einer softwaretechnischen Implementierung kann die Berechnungseinheit als Computerprogrammprodukt, als eine Funktion, als eine Routine, als Teil eines Programmcodes oder als ausführbares Objekt ausgebildet sein. Ein Computerprogrammprodukt, wie z.B. ein Computerprogramm-Mittel, kann beispielsweise als Speichermedium, wie z.B. Speicherkarte, USB-Stick, CD-ROM, DVD, oder auch in Form einer herunterladbaren Datei von einem Server in einem Netzwerk bereitgestellt oder geliefert werden. Dies kann zum Beispiel in einem drahtlosen Kommunikationsnetzwerk durch die Übertragung einer entsprechenden Datei mit dem Computerprogrammprodukt oder dem Computerprogramm-Mittel erfolgen.

Gemäß einer weiteren Ausführungsform des wasserführenden Haushaltsgeräts ist ein Temperatursensor zum Ermitteln einer Flüssigkeitstemperatur der durch den Rohrleitungsabschnitt geleiteten Flüssigkeit vorgesehen, wobei die Berechnungseinheit ferner zum Berechnen des lonenprofils der Flüssigkeit in Abhängigkeit der ermittelten Flüssigkeitstemperatur eingerichtet ist.

Die Flüssigkeitstemperatur beeinflusst sowohl die Mobilität der geladenen Teilchen in der Flüssigkeit als auch beispielsweise den Brechungsindex und die Leitfähigkeit. Um eine möglichst genaue Bestimmung des lonenprofils zu gewährleisten, ist ein Temperatursensor vorteilhaft, da dann die aktuelle tatsächliche Temperatur der Flüssigkeit bei der Bestimmung des lonenprofils berücksichtigt werden kann.

Gemäß einer weiteren Ausführungsform des wasserführenden Haushaltsgeräts ist der Temperatursensor als ein integrierter optischer Sensor ausgebildet.

Gemäß einer weiteren Ausführungsform des wasserführenden Haushaltsgeräts ist ein Strömungssensor zum Ermitteln einer Strömungsgeschwindigkeit der Flüssigkeit in dem Teilstück des Rohrleitungsabschnitts vorgesehen, wobei die Berechnungseinheit ferner zum Berechnen des lonenprofils der Flüssigkeit in Abhängigkeit der ermittelten Strömungsgeschwindigkeit eingerichtet ist.

Die Strömungsgeschwindigkeit hat einen Einfluss auf das sich ergebende lonenprofil in Abhängigkeit der Position entlang der Messstrecke, da die Strömungsgeschwindigkeit direkt bestimmt, wie lange die Flüssigkeit der Einwirkung des elektrischen Felds ausgesetzt war, wenn eine bestimmte Position entlang der Messstrecke erreicht ist. Um genaue Ergebnisse zu erhalten, ist die Kenntnis der Strömungsgeschwindigkeit daher vorteilhaft. Der Strömungssensor ist beispielsweise als ein Staudrucksensor, ein Differenzdrucksensor und/oder als ein Ultraschallsensor ausgebildet.

Gemäß einer weiteren Ausführungsform des wasserführenden Haushaltsgeräts sind die Elektroden so ausgebildet, dass sich ein im Wesentlichen homogenes elektrisches Feld oder ein homogenes elektrisches Feld im Bereich der Messstrecke ergibt.

Bei zwei planparallelen Metallplatten bildet sich zwischen den Platten ein derartiges homogenes elektrisches Feld aus. Lediglich in den Randbereichen können sich Inhomogenitäten ergeben. Die Verwendung von derartigen Elektroden vereinfacht die dem Modell zugrundeliegenden Berechnungen erheblich. Es ist aber auch denkbar, dass andere Elektroden, welche inhomogene elektrische Feldverläufe ergeben, verwendet werden.

Gemäß einer weiteren Ausführungsform des wasserführenden Haushaltsgeräts ist eine einstellbare Spannungsquelle zum Erzeugen des elektrischen Feldes vorgesehen.

Damit lässt sich die Sensorvorrichtung vorteilhaft an unterschiedliche Bedingungen anpassen und/oder Messungen bei unterschiedlichen elektrischen Feldstärken durchführen, was eine Bestimmungsgenauigkeit des lonenprofils erhöhen kann.

Gemäß einer weiteren Ausführungsform des wasserführenden Haushaltsgeräts ist die Flüssigkeit Wasser.

Gemäß einer weiteren Ausführungsform des wasserführenden Haushaltsgeräts umfasst das lonenprofil des Wassers eine lonenkonzentration für Calciumionen und eine lonenkonzentration für Magnesiumionen.

Calciumionen und Magnesiumionen sind die zwei wichtigsten sogenannten Härtebildner. Diese Ionen liegen in Wasser als zweifach positiv geladene Kationen vor. Ein Grad deutscher Härte (1° dH) entspricht dabei einer Gesamtionenkonzentration von ca. 0,1783 mmol/l. Leitungswasser hat in Deutschland regional unterschiedliche Härtegrade. In den meisten Regionen liegt die Gesamthärte im Bereich von 0° dH bis etwa 30° dH.

Gemäß einer weiteren Ausführungsform des wasserführenden Haushaltsgeräts ist das Haushaltsgerät als eine Geschirrspülmaschine, eine Waschmaschine, ein Dampfgarer, eine Kaffeemaschine, ein Wasserkocher, ein Wassererhitzer, ein Warmwasserspeicher und/oder eine Enthärtungsanlage ausgebildet.

Gemäß einem zweiten Aspekt wird ein Verfahren zum Betreiben eines wasserführenden Haushaltsgeräts mit einer Sensorvorrichtung zum Bestimmen eines lonenprofils einer Durchströmen mit der Flüssigkeit, eine erste Elektrode, eine zweite Elektrode und eine Anzahl von Sensorelementen auf. Dabei sind die erste Elektrode, die zweite Elektrode und der Rohrleitungsabschnitt derart relativ zueinander angeordnet, dass sich durch Anlegen einer Spannung zwischen der ersten Elektrode und der zweiten Elektrode ein elektrisches Feld in zumindest einem Teilstück des Rohrleitungsabschnitts ausbildet, wobei die Sensorelemente an einem Rand des Rohrleitungsabschnitts angeordnet sind und zum Ermitteln einer lokalen Flüssigkeitseigenschaft der durch den Rohrleitungsabschnitt geleiteten Flüssigkeit eingerichtet sind. Das Verfahren umfasst die Schritte:
- Durchströmen des Rohrleitungsabschnitts mit der Flüssigkeit,
- Anlegen der Spannung zwischen der ersten Elektrode und der zweiten Elektrode,
- Ermitteln der lokalen Flüssigkeitseigenschaft mit jedem der Sensorelemente, und
- Bestimmen des lonenprofils der Flüssigkeit in Abhängigkeit der ermittelten lokalen Flüssigkeitseigenschaft und des elektrischen Feldes.

Dieses Verfahren ermöglicht es vorteilhaft, jederzeit das lonenprofil der Flüssigkeit schnell und exakt bestimmen zu können. Basierend hierauf kann auf Schwankungen beispielsweise einer Wasserhärte sofort reagiert werden und somit ein effizienter Einsatz von Spül- und Waschmitteln garantiert werden. Des Weiteren kann eine vorzeitige Alterung von wasserführenden Elementen, zum Beispiel durch Verkalken, verhindert werden.

Weiterhin wird ein Computerprogrammprodukt vorgeschlagen, welches auf einer programmgesteuerten Einrichtung die Durchführung des wie oben erläuterten Verfahrens veranlasst.

Die für die vorgeschlagene Vorrichtung beschriebenen Ausführungsformen und Merkmale gelten für das vorgeschlagene Verfahren entsprechend.

Gemäß einem dritten Aspekt wird eine Sensorvorrichtung zum Bestimmen eines lonenprofils einer Flüssigkeit vorgeschlagen. Die Sensorvorrichtung weist einen Rohrleitungsabschnitt zum Durchströmen mit der Flüssigkeit, eine erste Elektrode, eine zweite Elektrode und eine Anzahl von Sensorelementen auf. Die erste Elektrode, die zweite Elektrode und der Rohrleitungsabschnitt sind derart relativ zueinander angeordnet, dass sich durch Anlegen einer Spannung zwischen der ersten Elektrode und der zweiten Elektrode ein elektrisches Feld in zumindest einem Teilstück des Rohrleitungsabschnitts ausbildet. Die elektrisches Feld in zumindest einem Teilstück des Rohrleitungsabschnitts ausbildet. Die Sensorelemente sind zum Ermitteln einer lokalen Flüssigkeitseigenschaft zum Bestimmen des lonenprofils der durch den Rohrleitungsabschnitt geleiteten Flüssigkeit eingerichtet. Diese Sensorvorrichtung ist vielfältig einsetzbar. Beispielsweise ist die Sensorvorrichtung als Feldgerät bei der Überprüfung von natürlichen Gewässern, in der Biomedizin zur Überprüfung von flüssigen Proben wie Blut, in der Lebensmittelchemie oder auch in der Prozesswasserüberwachung in der industriellen Produktion einsetzbar.

Im Vergleich mit herkömmlichen Sensorvorrichtungen ergeben sich die folgenden vorteilhaften Eigenschaften der Sensorvorrichtung: eine kurze Messdauer, eine lange Lebensdauer, es sind keine speziellen Materialen zur selektiven Funktionalisierung der Sensorelemente auf bestimmte lonensorten notwendig, an den Sensorelementen finden keine elektrochemischen Prozesse statt und ein Einfluss von leitfähigen Partikeln lässt sich leicht berücksichtigen und eliminieren.

Die zum ersten Aspekt beschriebenen Ausführungsformen der Sensorvorrichtung des wasserführenden Haushaltsgeräts gelten auch für die Sensorvorrichtung gemäß dem dritten Aspekt.

Weitere vorteilhafte Ausgestaltungen und Aspekte der Erfindung sind Gegenstand der Unteransprüche sowie der im Folgenden beschriebenen Ausführungsbeispiele der Erfindung. Im Weiteren wird die Erfindung anhand von bevorzugten Ausführungsformen unter Bezugnahme auf die beigelegten Figuren näher erläutert.
- Fig. 1: zeigt eine schematische perspektivische Ansicht einer Ausführungsform eines wasserführenden Haushaltsgeräts;
- Fig. 2: zeigt einen schematischen Querschnitt einer ersten Ausführungsform einer Sensorvorrichtung;
- Fig. 3: zeigt einen schematischen Querschnitt einer zweiten Ausführungsform einer Sensorvorrichtung;
- Fig. 4: zeigt ein Diagramm von Brechungsindexverläufen über der x-Achse der Fig. 3;
- Fig. 5: zeigt für mehrere Positionen entlang der x-Achse der Fig. 3 Diagramme von Konzentrations- und Brechungsindexprofilen über der y-Achse;
- Fig. 6: zeigt eine schematische perspektivische Ansicht einer dritten Ausführungsform einer Sensorvorrichtung;
- Fig. 7: zeigt eine schematische perspektivische Ansicht einer vierten Ausführungsform einer Sensorvorrichtung; und
- Fig. 8: zeigt ein beispielhaftes Blockschaltbild eines Verfahrens zum Betreiben eines wasserführenden Haushaltsgeräts.

In den Figuren sind gleiche oder funktionsgleiche Elemente mit denselben Bezugszeichen versehen worden, sofern nichts anderes angegeben ist.

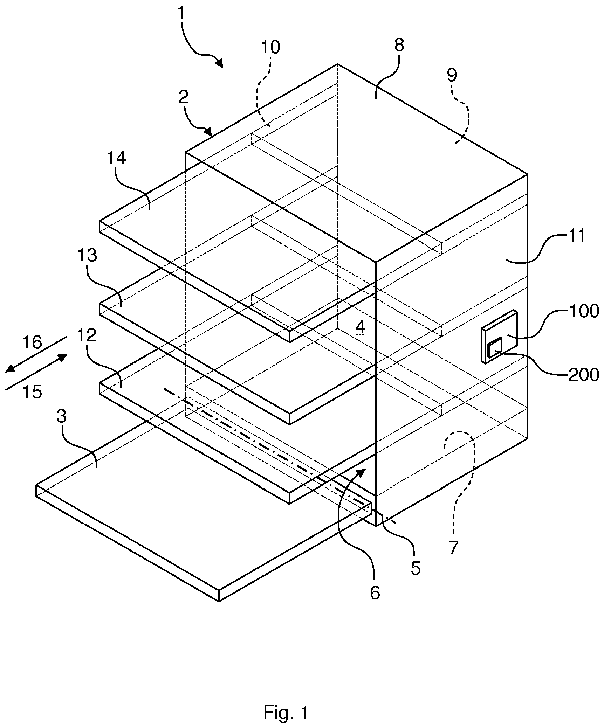

Die Fig. 1 zeigt eine schematische perspektivische Ansicht einer Ausführungsform eines wasserführenden Haushaltsgeräts 1, das hier als eine Haushalts-Geschirrspülmaschine ausgebildet ist. Die Haushalts-Geschirrspülmaschine 1 umfasst einen Spülbehälter 2, der durch eine Tür 3, insbesondere wasserdicht verschließbar ist. Hierzu kann zwischen der Tür 3 und dem Spülbehälter 2 eine Dichteinrichtung vorgesehen sein. Der Spülbehälter 2 ist vorzugsweise quaderförmig. Der Spülbehälter 2 kann in einem Gehäuse der Haushalts-Geschirrspülmaschine 1 angeordnet sein. Der Spülbehälter 2 und die Tür 3 können eine Spülkammer 4 zum Spülen von Spülgut bilden.

Die Tür 3 ist in der Fig. 1 in ihrer geöffneten Stellung dargestellt. Durch ein Schwenken um eine an einem unteren Ende der Tür 3 vorgesehene Schwenkachse 5 kann die Tür 3 geschlossen oder geöffnet werden. Mit Hilfe der Tür 3 kann eine Beschickungsöffnung 6 des Spülbehälters 2 geschlossen oder geöffnet werden. Der Spülbehälter 2 weist einen Boden 7, eine dem Boden 7 gegenüberliegend angeordnete Decke 8, eine der geschlossenen Tür 3 gegenüberliegend angeordnete Rückwand 9 und zwei einander gegenüberliegend angeordnete Seitenwände 10, 11 auf. Der Boden 7, die Decke 8, die Rückwand 9 und die Seitenwände 10, 11 können beispielsweise aus einem Edelstahlblech gefertigt sein. Alternativ kann beispielsweise der Boden 7 aus einem Kunststoffmaterial gefertigt sein.

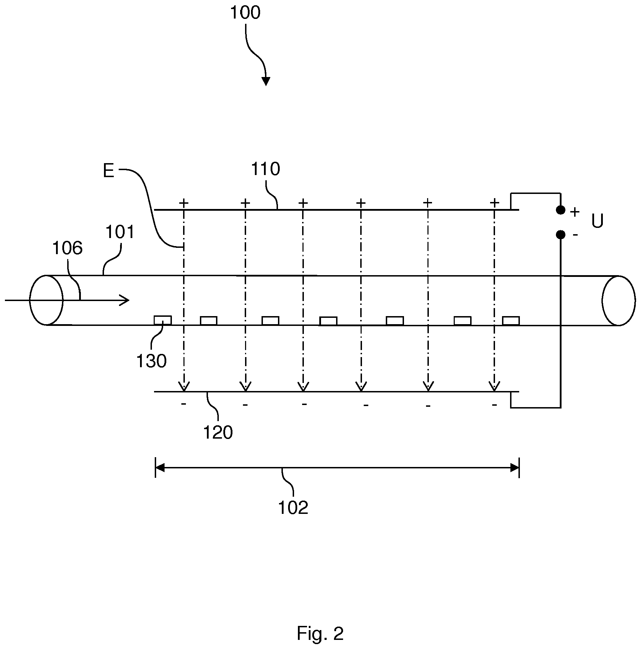

An der Seitenwand 11 ist in diesem Ausführungsbeispiel eine Sensorvorrichtung 100 zum Bestimmen eines lonenprofils des der Haushalts-Geschirrspülmaschine 1 zugeführten Wassers angeordnet. In anderen Ausführungsbeispielen kann die Sensorvorrichtung 100 auch an anderen Positionen des wasserführenden Haushaltsgeräts 1 angeordnet sein, vorzugsweise zum Beispiel in der Nähe einer Wasserzuführung (nicht dargestellt), um einen Aufwand zum Anschluss der Sensorvorrichtung 100 an die Zuleitung gering zu halten. Die Sensorvorrichtung 100 weist in diesem Beispiel auch eine Berechnungseinheit 200 auf, welche hier hardwaremäßig implementiert ist. Die Berechnungseinheit 200 ist dazu eingerichtet, das lonenprofil des der Geschirrspülmaschine 1 zugeführten Wassers mittels von Sensorelementen 130 (siehe z.B. Fig. 2, 3, 6, 7) ermittelten Messdaten zu bestimmen.

Die Haushalts-Geschirrspülmaschine 1 weist ferner zumindest eine Spülgutaufnahme 12 bis 14 auf. Vorzugsweise können mehrere, beispielsweise drei, Spülgutaufnahmen 12 bis 14 vorgesehen sein, wobei die Spülgutaufnahme 12 eine untere Spülgutaufnahme oder ein Unterkorb, die Spülgutaufnahme 13 eine obere Spülgutaufnahme oder ein Oberkorb und die Spülgutaufnahme 14 eine Besteckschublade sein kann. Wie die Fig. 1 weiterhin zeigt, sind die Spülgutaufnahmen 12 bis 14 übereinander in dem Spülbehälter 2 angeordnet. Jede Spülgutaufnahme 12 bis 14 ist wahlweise in den Spülbehälter 2 hinein- oder aus diesem herausverlagerbar. Insbesondere ist jede Spülgutaufnahme 12 bis 14 in einer Einschubrichtung 15 in den Spülbehälter 2 hineinschiebbar und entgegen der Einschubrichtung 15 in einer Auszugsrichtung 16 aus dem Spülbehälter 2 herausziehbar. Die untere Spülgutaufnahme 12 ist ferner mit einer später noch zu erläuternden Hebeeinrichtung von einer Ausgangsposition in eine Endposition, in der sie vorzugsweise vor der oberen Spülgutaufnahme 13 und auf derselben Höhe wie diese angeordnet ist, anhebbar.

Fig. 2 zeigt einen schematischen Querschnitt einer ersten Ausführungsform einer Sensorvorrichtung 100, die beispielsweise in der Haushalts-Geschirrspülmaschine 1 verwendet werden kann. Die Sensorvorrichtung 100 weist einen Rohrleitungsabschnitt 101 auf, der hier als ein rundes Kunststoffrohr ausgebildet ist. Das Kunststoffrohr 101 weist ein Teilstück 102 auf, welches zwischen zwei Elektroden 110, 120 verläuft. Die Elektroden 110, 120 sind als leitfähige Metallplatten ausgebildet. Die erste Elektrode 110 ist an den Plus-Pol einer Gleichspannungsquelle U angeschlossen und die zweite Elektrode 120 ist an den Minus-Pol der Gleichspannungsquelle U angeschlossen. Daher weist die Metallplatte 110 eine positive Ladung auf und die Metallplatte 120 weist eine negative Ladung auf, was jeweils durch entsprechende Symbole gekennzeichnet ist. Die Spannung der Gleichspannungsquelle U beträgt beispielsweise zwischen 10 V und 100 V und kann auch veränderbar sein. Aufgrund dieser Anordnung bildet sich zwischen den Metallplatten 110, 120 ein elektrisches Feld E aus. In dem Bereich zwischen den Metallplatten 110, 120 ist das elektrische Feld E nahezu homogen, das heißt es ist an jeder Position gleich stark und gleichgerichtet. Das elektrische Feld E durchdringt auch das Innere des Kunststoffrohrs 101. Das Teilstück 102 des Kunststoffrohrs 101, welches auch als Messstrecke bezeichnet werden kann, ist im Wesentlichen durch die Umfassung durch die Elektroden 110, 120 definiert.

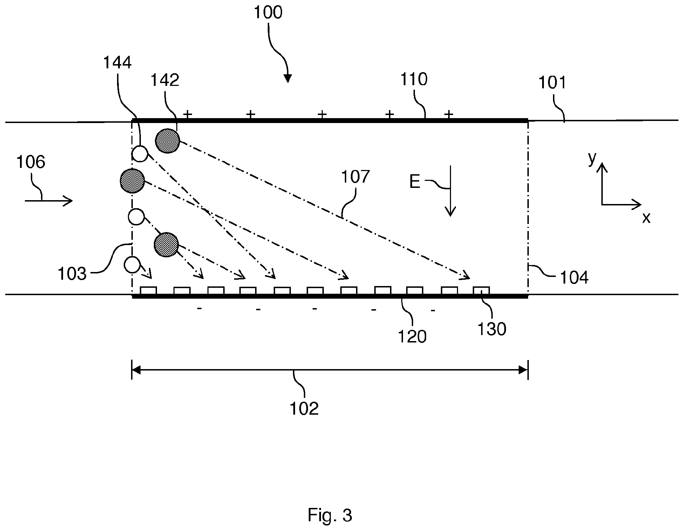

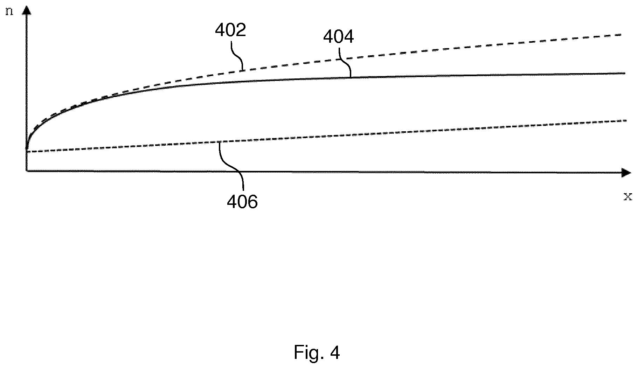

Auf der Seite der negativ geladenen Metallplatte 120 sind im Bereich der Messstrecke 102 eine Anzahl an Sensorelementen 130 angeordnet, von denen der Übersicht halber nur das Erste mit einem Bezugszeichen versehen ist. Die Sensorelemente 130 sind vorliegend als integrierte optische Elemente ausgebildet und zur Bestimmung des Brechungsindex n (siehe Fig. 4) an ihrer zum Inneren des Kunststoffrohrs 101 hin weisenden Oberfläche eingerichtet. Ein solcher Brechungsindexsensor 130 weist beispielsweise einen Durchmesser von 50 - 100 µm, beispielsweise 60 µm auf. Im Betrieb der Sensorvorrichtung 100 strömt die Flüssigkeit gemäß dem Strömungsvektor 106 durch das Kunststoffrohr 101. Die Strömungsgeschwindigkeit liegt beispielsweise in einem Bereich von bis zu 10 mm/s, beispielsweise 2 mm/s. Nicht dargestellt sind Anschlussstutzen zum Anschluss des Kunststoffrohrs 101 an den Flüssigkeitskreislauf, dessen Flüssigkeit untersucht werden soll. Die genauere Funktionsweise der Sensorvorrichtung 100 wird im Zusammenhang mit der Fig. 3 näher erläutert.

Fig. 3 zeigt einen schematischen Querschnitt einer zweiten Ausführungsform der Sensorvorrichtung 100, die sich von der in Fig. 2 gezeigten Ausführungsform darin unterscheidet, dass hier die Elektroden 110, 120 direkt an das Kunststoffrohr 101 angrenzen. Die weiteren Merkmale sind identisch.

Der Fokus der Fig. 3 liegt auf der Darstellung einer Bewegungsrichtung 107 von in der Flüssigkeit enthaltenen Ionen 142, 144. Vorliegend ist die Flüssigkeit Wasser und die Ionen sind Calciumionen 142 und Magnesiumionen 144. Ferner ist mit einer gestrichelten Linie eine gedachte Einlassöffnung 103 sowie eine entsprechende Auslassöffnung 104 der Messstrecke 102 eingezeichnet. Zur Festlegung von Richtungen ist ein Koordinatensystem mit einer x-Achse und einer y-Achse dargestellt. Die x-Achse verläuft hier parallel zu dem Strömungsvektor 106 der Flüssigkeit sowie zu den Elektroden 110, 120 und die y-Achse verläuft parallel zu dem elektrischen Feld E.

Sobald die Flüssigkeit mit dem Ionen 142, 144 über die Einlassöffnung 103 in den Bereich der Messstrecke 102 einströmt, wirkt das elektrische Feld E auf die Ionen 142, 144 ein und beschleunigt sie in Richtung zu der gegenpoligen Elektrode. Da die Calciumionen 142 und die Magnesiumionen 144 Kationen und damit positiv geladen sind, werden sie zur negativ geladen Elektrode 120 hin beschleunigt. Die Flüssigkeit bremst die Bewegung der Ionen 142, 144, wodurch sich eine im Wesentlichen konstante Geschwindigkeit der Ionen 142, 144 in Richtung des elektrischen Feldes E ergibt. Der Betrag der Geschwindigkeit hängt dabei von einem lonenradius, einer Ladungszahl und der Stärke des elektrischen Feldes E ab. Da die Flüssigkeit gemäß dem Strömungsvektor 106 mit einer gewissen Strömungsgeschwindigkeit weiter strömt, bewegen sich die Ionen 142, 144 in die eingezeichnete Richtung 107. Diese Richtung 107 entspricht der überlagerten Bewegung entlang dem Strömungsfeld der Flüssigkeit und der aufgrund der elektrostatischen Anziehung bewirkten Bewegung entlang dem elektrischen Feld E.

Die kleineren Magnesiumionen 144 bewegen sich aufgrund des elektrischen Feldes E schneller hin zur Elektrode 120 als die größeren Calciumionen 142. Deshalb weist die effektive Bewegungsrichtung 107 der Magnesiumionen stärker hin zu der zweiten Elektrode 120. Im Ergebnis bildet sich eine stationäre lonenverteilung für die Magnesiumionen 144 schneller aus als für die Calciumionen 142.

Die Sensorelemente 130 messen an unterschiedlichen Positionen entlang der x-Achse den lokalen Brechungsindex n der Flüssigkeit. Das Ergebnis einer solchen Messung ist in Fig. 4 gezeigt.

Fig. 4 zeigt ein Diagramm mit drei Brechungsindexverläufen entlang der x-Achse (siehe Fig. 3). Die Kurve 402 zeigt dabei den Brechungsindexverlauf, der sich bei einer Messung mit einer Sensorvorrichtung 100 beispielsweise gemäß der Fig. 2 oder Fig. 3 ergeben könnte. Es ist in der Fig. 4 erkennbar, dass die Kurve 402 einer Summe der Kurven 404 und 406 entspricht. Die Kurven 404 und 406 zeigen den theoretischen Brechungsindexverlauf, der sich ergeben würde, wenn jeweils nur eine Ionensorte in der Flüssigkeit vorhanden wäre. Die Kurve 404 entspricht hierbei dem Effekt von sehr kleinen und/oder stark geladenen Ionen, welche sehr schnell ihre Gleichgewichtsverteilung einnehmen, was daran erkennbar ist, dass die Kurve 404 schnell abflacht. Demgegenüber zeigt die Kurve 406 einen in dem dargestellten Bereich fast linearen Anstieg, was zeigt, dass die dafür verantwortliche Ionensorte sich in der Flüssigkeit nur langsam bewegen kann. Der Grund hierfür kann ein großer lonenradius und/oder eine geringe Ladung sein.

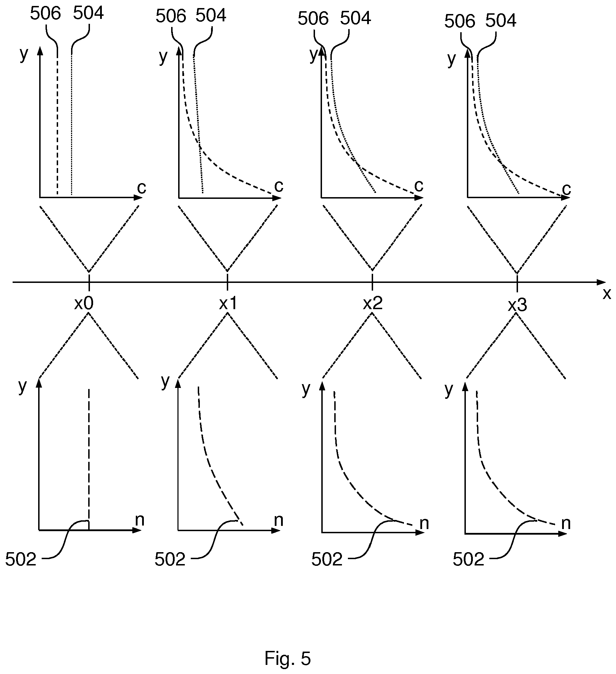

Fig. 5 zeigt für mehrere Positionen x0, x1, x2, x3 entlang der x-Achse Diagramme von Konzentrations- und Brechungsindexprofilen über der y-Achse. Die übereinander dargestellten Diagramme stellen jeweils die Verläufe dar, wie sie sich in y-Richtung an der jeweiligen x-Position ergeben. Die x- und y-Achse entsprechen den in Fig. 3 definierten Richtungen. Die obere Reihe von Diagrammen zeigt die Konzentrationsprofile 504, 506 für zwei unterschiedliche Ionensorten, beispielsweise Calciumionen 142 und Magnesiumionen 144. Die untere Reihe von Diagrammen zeigt das sich ausbildende Brechungsindexprofil 502 in Richtung der y-Achse.

Position x0 entspricht beispielsweise der Einlassöffnung 103 der Messstrecke 102 (siehe Fig. 2 und 3). An dieser Position sind die Ionen in der Flüssigkeit noch gleichmäßig verteilt, weshalb die Konzentration c entlang der y-Achse konstant ist. Hieraus ergibt sich ebenfalls ein konstanter Brechungsindex n entlang der y-Achse. Ab dieser Position befinden sich die Ionen unter dem Einfluss des elektrischen Feldes E (siehe Fig. 2 und 3).

Bei Position x1 hat sich für die sehr gut beweglichen Ionen bereits eine Gleichgewichtsverteilung eingestellt, wie sich aus einem Vergleich mit der Verteilung 506 an der nachfolgenden Position x2 ergibt. Die Verteilung 504 der langsameren Ionen hat sich hingegen noch nicht stark verändert. Der Brechungsindexverlauf 502 entlang der y-Achse zeigt den kombinierten Effekt beider Ionensorten.

Bei Position x2 hat sich nun auch für die langsameren Ionen eine Gleichgewichtsverteilung ausgebildet, wie sich aus einem Vergleich mit der Verteilung 504 an der nachfolgenden Position x3 ergibt. Der Brechungsindexverlauf 502 hat sich daher ebenfalls weiter verändert.

Bei Position x3 ergeben sich schließlich gegenüber Position x2 unveränderte lonenkonzentrationsprofile 504, 506 sowie der gleiche Brechungsindexverlauf 502. Dies zeigt, dass alle Ionen in ihrer jeweiligen Gleichgewichtsverteilung sind. Die Messung ist damit abgeschlossen.

Durch mathematische Modellierung unter der Verwendung von beispielsweise Eichmessungen und/oder vereinfachenden Annahmen, lässt sich aus den gemessenen Brechungsindexverläufen 502 auf die lonensorten und die unterschiedlichen Ionenkonzentrationsprofile 504, 506 schließen.

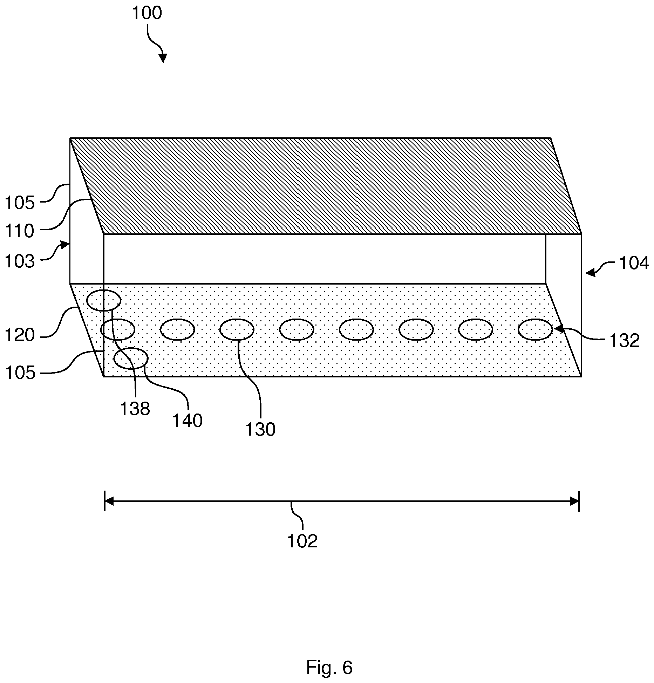

Fig. 6 zeigt eine schematische perspektivische Ansicht einer dritten Ausführungsform einer Sensorvorrichtung 100. Die Sensorvorrichtung 100 der Fig. 6 unterscheidet sich vor allem in ihrem geometrischen Aufbau von den Sensorvorrichtungen der Fig. 2 und 3, da hier ein rechteckiger Querschnitt im Bereich der Messstrecke 102 gewählt ist. Der Elektrodenabstand beträgt in diesem Beispiel 0,5 mm, der Abstand der Seitenwände 105 zueinander beträgt 1 mm. Aus Gründen der Übersicht ist auf die Darstellung eines Rohrleitungsabschnitts 101 sowie einer Spannungsquelle U verzichtet (siehe Fig. 2, 3).

Bei der Sensorvorrichtung 100 der Fig. 6 sind auf der Seite der zweiten Elektrode 120 eine Anzahl von Sensorelementen 130 angeordnet, die hier eine lineare Messreihe 132 bilden. Mittels der linearen Messreihe 132 lässt sich ein Brechungsindexverlauf 402 (siehe Fig. 4) entlang der Strömungsrichtung 106 bestimmen, woraus sich mittels mathematischer Modellierung das lonenprofil der Flüssigkeit bestimmen lässt.

Darüber hinaus weist die Sensorvorrichtung 100 einen an der zweiten Elektrode 120 angeordneten Temperatursensor 138 und einen Strömungssensor 140 auf, um die Temperatur und die Strömungsgeschwindigkeit der Flüssigkeit aktuell bestimmen zu können.

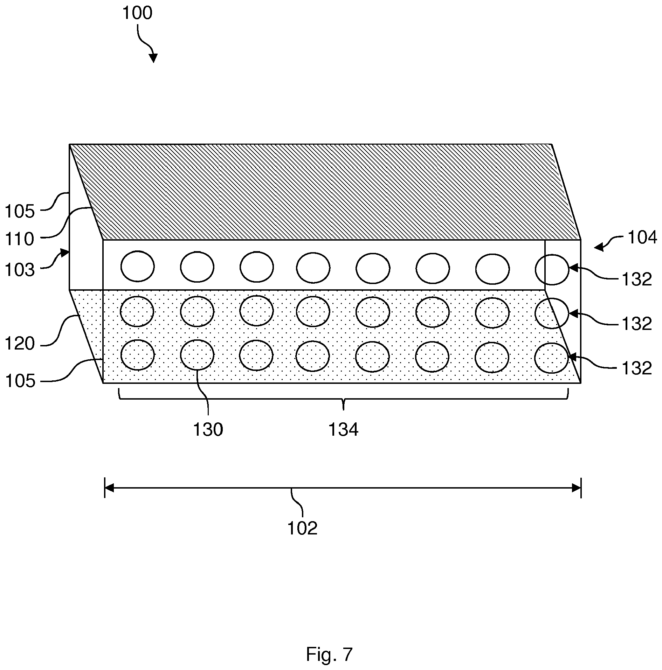

Fig. 7 zeigt eine schematische perspektivische Ansicht einer vierten Ausführungsform einer Sensorvorrichtung 100. Die dargestellte Ausführungsform entspricht der in der Fig. 6 gezeigten Ausführungsform, mit dem Unterschied, dass anstelle einer linearen Messreihe 132 drei solcher Messreihen 132 an einer seitlichen Wand 105, die parallel zu dem elektrischen Feld E (siehe Fig. 2, 3) verläuft, angeordnet sind. Die Sensorelemente 130 bilden hier eine Sensormatrix 134. Aus den Spalten dieser Sensormatrix 134 lassen sich beispielsweise Brechungsindexverläufe 502 entlang der y-Achse, wie sie in der Fig. 5 dargestellt sind, bestimmen. Diese Ausführungsform ist für detaillierte Modelle besonders vorteilhaft, da mit ihr eine große Zahl an Daten ermittelbar ist, welche eine genaue Bestimmung des lonenprofils verbessern.

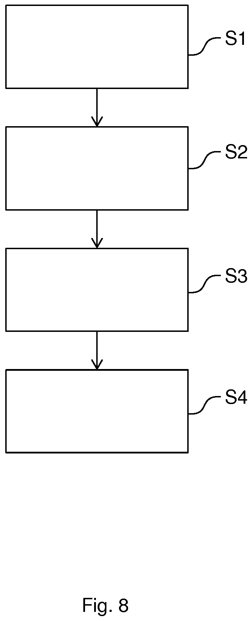

Fig. 8 zeigt ein beispielhaftes Blockschaltbild eines Ausführungsbeispiels eines Verfahrens zum Betreiben eines wasserführenden Haushaltsgeräts 1, beispielsweise der in Fig. 1 gezeigten Geschirrspülmaschine, mit der Sensorvorrichtung 100 zum Bestimmen eines lonenprofils einer Flüssigkeit. Die Sensorvorrichtung 100 weist einen Rohrleitungsabschnitt 101 zum Durchströmen mit der Flüssigkeit, eine erste Elektrode 110, eine zweite Elektrode 120 und eine Anzahl von Sensorelementen 130 aufweist (siehe Fig. 2, 3, 6, 7). Die erste Elektrode 110, die zweite Elektrode 120 und der Rohrleitungsabschnitt 101 sind derart relativ zueinander angeordnet, dass sich durch Anlegen einer Spannung U zwischen der ersten Elektrode 110 und der zweiten Elektrode 120 ein elektrisches Feld E in zumindest einem Teilstück 102 des Rohrleitungsabschnitts 101 ausbildet (siehe Fig. 2, 3). Die Sensorelemente 130 sind zum Ermitteln einer lokalen Flüssigkeitseigenschaft der durch den Rohrleitungsabschnitt 101 geleiteten Flüssigkeit eingerichtet. Das Verharen der Fig. 8 umfasst die folgenden Schritte S1 bis S4:
In Schritt S1 wird der Rohrleitungsabschnitt 101 der Sensorvorrichtung 100 mit der Flüssigkeit durchströmt. In Schritt S2 wird die Spannung U zwischen der ersten Elektrode 110 und der zweiten Elektrode 120 angelegt. In Schritt S3 wird die lokale Flüssigkeitseigenschaft mit jedem der Sensorelemente 130 ermittelt. In Schritt S4 wird das lonenprofil der Flüssigkeit in Abhängigkeit der ermittelten lokalen Flüssigkeitseigenschaft und des elektrischen Feldes E ermittelt.

Das Durchströmen S1 des Rohrleitungsabschnitts 101 der Sensorvorrichtung 100 mit der Flüssigkeit wird beispielsweise bewerkstelligt, indem ein entsprechendes Einlassventil geöffnet wird. Bevorzugt wird dies so vorgenommen, dass die Strömungsgeschwindigkeit 106 der Flüssigkeit in dem Teilstück 102 bekannt und konstant ist. Ferner ist es vorteilhaft, wenn sich dabei eine laminare Flüssigkeitsströmung ausbildet, was durch ein entsprechendes Design, insbesondere bezüglich der geometrischen Abmessungen und Form, erreicht werden kann.

Die Spannung U wird von einer Gleichspannungsquelle bereitgestellt und liegt beispielsweise in einem Bereich von 10 - 100 V.

Zum Ermitteln S3 der lokalen Flüssigkeitseigenschaft mittels der Sensorelemente 130 sind diese beispielsweise mit einer entsprechenden Elektronik verbunden. Die Elektronik steuert die Sensorelemente derart an, dass diese eine Messung durchführen. Ebenso kann das Ergebnis der Messung von der Elektronik erfasst werden. Das geschieht beispielsweise durch ein Erfassen eines elektrischen Signals, das von einem Detektor eines Sensorelements 130 erzeugt wird. Die vorstehenden Angaben sind lediglich beispielhaft zu verstehen und hängen von der Art der Sensorelemente 130 ab.

Das Bestimmen S4 des lonenprofils der Flüssigkeit erfolgt insbesondere durch eine dafür vorgesehene Berechnungseinheit 200. Diese ist beispielsweise als ein Prozessor oder ein Computer implementiert. Ferner kann die Berechnungseinheit 200 als eine Routine, eine Applikation, eine Software und/oder ein Programm implementiert sein, welches auf einer weiteren Steuerungseinheit, beispielsweise einer Steuerungseinheit des Geschirrspülmaschine 1, ausführbar ist.

In Ausführungsformen des Verfahrens werden beispielsweise weitere Parameter, wie die Flüssigkeitstemperatur und/oder die Strömungsgeschwindigkeit der Flüssigkeit, durch entsprechende Sensoren 138, 140 (siehe Fig. 6) ermittelt und bei der Bestimmung des lonenprofils entsprechend als Parameter berücksichtigt.

Obwohl die vorliegende Erfindung anhand von Ausführungsbeispielen beschrieben wurde, ist sie vielfältig modifizierbar.

### Verwendete Bezugszeichen:

- 1: Wasserführendes Haushaltsgerät
- 2: Spülbehälter
- 3: Tür
- 4: Spülkammer
- 5: Schwenkachse
- 6: Beschickungsöffnung
- 7: Boden
- 8: Decke
- 9: Rückwand
- 10: Seitenwand
- 11: Seitenwand
- 12: Spülgutaufnahme
- 13: Spülgutaufnahme
- 14: Spülgutaufnahme
- 15: Einschubrichtung
- 16: Auszugsrichtung
- 100: Sensorvorrichtung
- 101: Rohrleitungsabschnitt
- 102: Teilstück
- 103: Einlassöffnung
- 104: Auslassöffnung
- 105: Seitenwand
- 106: Strömungsvektor
- 107: lonenbewegungsrichtung
- 110: erste Elektrode
- 120: zweite Elektrode
- 130: Sensorelement
- 132: Reihe von Sensorelementen
- 134: Matrix von Sensorelementen
- 138: Temperatursensor
- 140: Strömungssensor
- 142: Calciumion
- 144: Magnesiumion
- 200: Berechnungseinheit
- 402: lokaler Brechungsindexverlauf insgesamt
- 404: lokaler Brechungsindexverlauf
- 406: lokaler Brechungsindexverlauf
- 502: Brechungsindexverlauf entlang y-Richtung
- 504: Konzentrationsprofil erste Ionensorte entlang y-Richtung
- 506: Konzentrationsprofil zweite Ionensorte entlang y-Richtung

- c: Stoffkonzentration
- E: elektrisches Feld/Feldlinien
- n: Brechungsindex
- S1: Vefahrensschritt
- S2: Vefahrensschritt
- S3: Vefahrensschritt
- S4: Vefahrensschritt
- U: Spannungsquelle
- x0: Position
- x1: Position
- x2: Position
- x3: Position

## Patentansprüche

1. Wasserführendes Haushaltsgerät (1), insbesondere Geschirrspülmaschine, mit einer Sensorvorrichtung (100) zum Bestimmen eines lonenprofils einer Flüssigkeit, wobei das lonenprofil eine Information zu der Konzentration von unterschiedlichen in der Flüssigkeit enthaltenen lonensorten umfasst, wobei die Sensorvorrichtung (100) einen Rohrleitungsabschnitt (101) zum Durchströmen mit der Flüssigkeit, eine erste Elektrode (110), eine zweite Elektrode (120) und eine Anzahl von Sensorelementen (130) aufweist, wobei die erste Elektrode (110), die zweite Elektrode (120) und der Rohrleitungsabschnitt (101) derart relativ zueinander angeordnet sind, dass sich durch Anlegen einer Spannung (U) zwischen der ersten Elektrode (110) und der zweiten Elektrode (120) ein elektrisches Feld (E) in zumindest einem Teilstück (102) des Rohrleitungsabschnitts (101) ausbildet, und wobei die Sensorelemente (130) an einem Rand des Rohrleitungsabschnitts (101) angeordnet sind und zum Ermitteln einer lokalen Flüssigkeitseigenschaft zum Bestimmen des lonenprofils der durch den Rohrleitungsabschnitt (101) geleiteten Flüssigkeit eingerichtet sind.

2. Wasserführendes Haushaltsgerät nach Anspruch 1, **dadurch gekennzeichnet, dass** die lokale Flüssigkeitseigenschaft eine lokale elektrische Leitfähigkeit ist und die Sensorelemente (130) zum Bestimmen der lokalen elektrischen Leitfähigkeit eingerichtet sind.

3. Wasserführendes Haushaltsgerät nach Anspruch 1, **dadurch gekennzeichnet, dass** die lokale Flüssigkeitseigenschaft ein lokaler Brechungsindex ist und die Sensorelemente (130) zum Bestimmen des lokalen Brechungsindex eingerichtet sind.

4. Wasserführendes Haushaltsgerät nach Anspruch 3, **dadurch gekennzeichnet, dass** die Sensorelemente (130) zum Bestimmen des lokalen Brechungsindex als integrierte optische Strukturen ausgebildet sind.

5. Wasserführendes Haushaltsgerät nach einem der Ansprüche 1 - 4, **dadurch gekennzeichnet, dass** die Sensorelemente (130) entlang einer Strömungsrichtung (106) der Flüssigkeit in dem Rohrleitungsabschnitt (101) angeordnet sind.

6. Wasserführendes Haushaltsgerät nach einem der Ansprüche 1 - 4, **dadurch gekennzeichnet, dass** die zweite Elektrode (120) als Kathode ausgebildet ist, wobei die Sensorelemente (130) entlang einer Strömungsrichtung (106) der Flüssigkeit in dem Rohrleitungsabschnitt (101) in einer Reihe (132) auf der Seite der Kathode angeordnet sind.

7. Wasserführendes Haushaltsgerät nach einem der Ansprüche 1 - 6, **dadurch gekennzeichnet, dass** die Sensorelemente (130) in einer Ebene parallel zu dem elektrischen Feld (108) zwischen der ersten Elektrode (110) und der zweiten Elektrode (120) seitlich in dem Rohrleitungsabschnitt (101) in einer Sensormatrix (134) angeordnet sind.

8. Wasserführendes Haushaltsgerät nacheinem der Ansprüche 1 - 7, **dadurch gekennzeichnet, dass** ferner eine Berechnungseinheit zum Berechnen des lonenprofils der Flüssigkeit in Abhängigkeit der ermittelten lokalen Flüssigkeitseigenschaft und dem elektrischen Feld (E) vorgesehen ist.

9. Wasserführendes Haushaltsgerät nach Anspruch 8, **dadurch gekennzeichnet, dass** ein Temperatursensor (138) zum Ermitteln einer Flüssigkeitstemperatur der durch den Rohrleitungsabschnitt (101) geleiteten Flüssigkeit vorgesehen ist, wobei die Berechnungseinheit (200) ferner zum Berechnen des lonenprofils der Flüssigkeit in Abhängigkeit der ermittelten Flüssigkeitstemperatur eingerichtet ist.

10. Wasserführendes Haushaltsgerät nach Anspruch 9, **dadurch gekennzeichnet, dass** der Temperatursensor (138) als ein integrierter optischer Sensor ausgebildet ist.

11. Wasserführendes Haushaltsgerät nach einem der Ansprüche 8 - 10, **dadurch gekennzeichnet, dass** ein Strömungssensor (140) zum Ermitteln einer Strömungsgeschwindigkeit (106) der Flüssigkeit in dem Teilstück (102) des Rohrleitungsabschnitts (101) vorgesehen ist, wobei die Berechnungseinheit (200) ferner zum Berechnen des lonenprofils der Flüssigkeit in Abhängigkeit der ermittelten Strömungsgeschwindigkeit (106) eingerichtet ist.

12. Wasserführendes Haushaltsgerät nach einem der Ansprüche 1 - 11, **dadurch gekennzeichnet, dass** die Flüssigkeit Wasser ist.

13. Wasserführendes Haushaltsgerät nach Anspruch 12, **dadurch gekennzeichnet, dass** das lonenprofil des Wassers eine lonenkonzentration für Calciumionen (142) und eine lonenkonzentration für Magnesiumionen (144) umfasst.

14. Wasserführendes Haushaltsgerät nach einem der Ansprüche 1 - 13, **dadurch gekennzeichnet, dass** das Haushaltsgerät als eine Geschirrspülmaschine, eine Waschmaschine, ein Dampfgarer, eine Kaffeemaschine, ein Wasserkocher, ein Wassererhitzer, ein Warmwasserspeicher und/oder eine Enthärtungsanlage ausgebildet ist.

15. Verfahren zum Betreiben eines wasserführenden Haushaltsgeräts (1) mit einer Sensorvorrichtung (100) zum Bestimmen eines lonenprofils einer Flüssigkeit, wobei das lonenprofil eine Information zu der Konzentration von unterschiedlichen in der Flüssigkeit enthaltenen lonensorten umfasst, wobei die Sensorvorrichtung (100) einen Rohrleitungsabschnitt (101) zum Durchströmen mit der Flüssigkeit, eine erste Elektrode (110), eine zweite Elektrode (120) und eine Anzahl von Sensorelementen (130) aufweist, wobei die erste Elektrode (110), die zweite Elektrode (120) und der Rohrleitungsabschnitt (101) derart relativ zueinander angeordnet sind, dass sich durch Anlegen einer Spannung (U) zwischen der ersten Elektrode (110) und der zweiten Elektrode (120) ein elektrisches Feld (E) in zumindest einem Teilstück (102) des Rohrleitungsabschnitts (101) ausbildet, und wobei die Sensorelemente (130) an einem Rand des Rohrleitungsabschnitts (101) angeordnet sind und zum Ermitteln einer lokalen Flüssigkeitseigenschaft der durch den Rohrleitungsabschnitt (101) geleiteten Flüssigkeit eingerichtet sind, mit den Schritten:
Durchströmen (S1) des Rohrleitungsabschnitts (101) mit der Flüssigkeit,
Anlegen (S2) der Spannung (U) zwischen der ersten Elektrode (110) und der zweiten Elektrode (120),
Ermitteln (S3) der lokalen Flüssigkeitseigenschaft mit jedem der Sensorelemente (130), und
Bestimmen (S4) des lonenprofils der Flüssigkeit in Abhängigkeit der ermittelten lokalen Flüssigkeitseigenschaft und des elektrischen Feldes (E).

## Claims

1. Water-conducting household appliance (1), in particular dishwasher, with a sensor apparatus (100) for determining an ion profile of a liquid, wherein the ion profile comprises an item of information relating to the concentration of different types of ion contained in the liquid, wherein the sensor apparatus (100) has a pipeline section (101) to allow the liquid to pass through, a first electrode (110), a second electrode (120) and a number of sensor elements (130), wherein the first electrode (110), the second electrode (120) and the pipeline section (101) are arranged relative to one another so that by applying a voltage (U) between the first electrode (110) and the second electrode (120), an electrical field (E) forms in at least one part (102) of the pipeline section (101), and wherein the sensor elements (130) are arranged on an edge of the pipeline section (101) and are designed to ascertain a local liquid property in order to determine the ion profile of the liquid routed through the pipeline section (101).

2. Water-conducting household appliance according to claim 1, **characterised in that** the local liquid property is a local electrical conductivity and the sensor elements (130) are designed to determine the local electrical conductivity.

3. Water-conducting household appliance according to claim 1, **characterised in that** the local liquid property is a local refractive index and the sensor elements (130) are designed to determine the local refractive index.

4. Water-conducting household appliance according to claim 3, **characterised in that** the sensor elements (130) are embodied to determine the local refractive index as integrated optical structures.

5. Water-conducting household appliance according to one of the preceding claims 1 - 4, **characterised in that** the sensor elements (130) are arranged along a flow direction (106) of the liquid in the pipeline section (101).

6. Water-conducting household appliance according to one of claims 1 - 4, **characterised in that** the second electrode (120) is embodied as a cathode, wherein the sensor elements (130) are arranged in a row (132) on the side of the cathode along a flow direction (106) of the liquid in the pipeline section (101).

7. Water-conducting household appliance according to one of claims 1 - 6, **characterised in that** in a plane parallel to the electrical field (108) between the first electrode (110) and the second electrode (120) the sensor elements (130) are arranged laterally in the pipeline section (101) in a sensor matrix (134).

8. Water-conducting household appliance according to one of claims 1 - 7, **characterised in that** a refractive unit for calculating the ion profile of the liquid is also provided as a function of the determined local liquid property and the electrical field (E).

9. Water-conducting household appliance according to claim 8, **characterised in that** a temperature sensor (138) is provided to determine a liquid temperature of the liquid routed through the pipeline section (101), wherein the refractive unit (200) is further designed to calculate the ion profile of the liquid as a function of the determined liquid temperature.

10. Water-conducting household appliance according to claim 9, **characterised in that** the temperature sensor (138) is embodied as an integrated optical sensor.

11. Water-conducting household appliance according to one of claims 8 - 10, **characterised in that** a flow sensor (140) is provided to ascertain a flow speed (106) of the liquid in the part (102) of the pipeline section (101), wherein the refractive unit (200) is further designed to calculate the ion profile of the liquid as a function of the determined flow speed (106).

12. Water-conducting household appliance according to one of claims 1 - 11, **characterised in that** the liquid is water.

13. Water-conducting household appliance according to claim 12, **characterised in that** the ion profile of the water comprises an ion concentration for calcium ion (142) and an ion concentration for magnesium ion (144).

14. Water-conducting household appliance according to one of claims 1 - 13, **characterised in that** the household appliance is embodied as a dishwasher, a washing machine, a steamer, a coffee machine, a kettle, a water heater, a hot water tank and/or a water softening system.

15. Method for operating a water-conducting household appliance (1) with a sensor apparatus (100) for determining an ion profile of a liquid, wherein the ion profile comprises an item of information relating to the concentration of different types of ion contained in the liquid, wherein the sensor apparatus (100) has a pipeline section (101) to allow the liquid to pass through, a first electrode (110), a second electrode (120) and a number of sensor elements (130), wherein the first electrode (110), the second electrode (120) and the pipeline section (101) are arranged relative to one another so that by applying a voltage (U) between the first electrode (110) and the second electrode (120), an electrical field (E) forms in at least one part (102) of the pipeline section (101), and wherein the sensor elements (130) are arranged on an edge of the pipeline section (101) and are designed to ascertain a local liquid property of the liquid routed through the pipeline section (101), having the steps:
allowing liquid to pass (SI) through the pipeline section (101),
applying (S2) the voltage between the first electrode (110) and the second electrode (120),
ascertaining (S3) the local liquid property with each of the sensor elements (130), and
determining (S4) the ion profile of the liquid as a function of the determined local liquid property and the electric field (E).

## Revendications

1. Appareil ménager à circulation d'eau (1), notamment machine à laver la vaisselle, comportant un dispositif capteur (100) pour capter un profil ionique d'un liquide, le profil ionique comportant une information concernant la concentration de différents types d'ions contenus dans le liquide, le dispositif capteur (100) comprenant une section de conduite (101) permettant le passage du liquide, une première électrode (110), une seconde électrode (120) et un certain nombre d'éléments de détection (130), la première électrode (110), la seconde électrode (120) et la section de conduite (101) étant disposées les unes par rapport aux autres de sorte que par suite de l'application d'une tension (U) entre la première électrode (110) et la seconde électrode (120), un champ électrique (E) se constitue dans au moins un tronçon (102) de la section de conduite (101), les éléments capteurs (130) étant disposés sur un bord de la section de conduite (101) et aptes à capter une propriété locale du liquide pour déterminer le profil ionique du liquide passant par la section de conduite (101).

2. Appareil ménager à circulation d'eau selon la revendication 1, **caractérisé en ce que** la propriété locale du liquide est une conductivité électrique locale et **en ce que** les éléments capteurs (130) sont conçus pour déterminer la conductivité électrique locale.

3. Appareil ménager à circulation d'eau selon la revendication 1, **caractérisé en ce que** la propriété locale du liquide est un indice de réfraction local et **en ce que** les éléments capteurs (130) sont conçus pour déterminer l'indice de réfraction local.

4. Appareil ménager à circulation d'eau selon la revendication 3, **caractérisé en ce que** les éléments capteurs (130) sont configurés comme des structures optiques intégrées pour déterminer l'indice de réfraction local.

5. Appareil ménager à circulation d'eau selon l'une des revendications 1 à 4, **caractérisé en ce que** les éléments capteurs (130) sont disposés selon un sens d'écoulement (106) du liquide dans la section de conduite (101).

6. Appareil ménager à circulation d'eau selon l'une des revendications 1 à 4, **caractérisé en ce que** la seconde électrode (120) est prévue sous forme de cathode, les éléments capteurs (130) étant disposés selon un sens d'écoulement (106) du liquide dans la section de conduite (101) en une rangée (132) du côté de la cathode.

7. Appareil ménager à circulation d'eau selon l'une des revendications 1 à 6, **caractérisé en ce que** les éléments capteurs (130) sont disposés dans un plan en parallèle au champ électrique (108) entre la première électrode (110) et la seconde électrode (120) latéralement dans la section de conduite (101) dans une matrice de capteurs (134).

8. Appareil ménager à circulation d'eau selon l'une des revendications 1 à 7, **caractérisé en ce que** par ailleurs, une unité de calcul pour calculer le profil ionique du liquide en fonction de la propriété locale du liquide relevée et du champ électrique (E) est prévue.

9. Appareil ménager à circulation d'eau selon la revendication 8, **caractérisé en ce qu'**un capteur de température (138) pour relever une température du liquide passant par la section de conduite (101) est prévu, l'unité de calcul (200) étant par ailleurs conçue pour calculer le profil ionique du liquide en fonction de la température de liquide relevée.

10. Appareil ménager à circulation d'eau selon la revendication 9, **caractérisé en ce que** le capteur de température (138) est conçu sous forme d'un capteur optique intégré.

11. Appareil ménager à circulation d'eau selon l'une des revendications 8 à 10, **caractérisé en ce qu'**un capteur d'écoulement (14) pour capter une vitesse d'écoulement (106) du liquide est prévu dans le tronçon (102) de la section de conduite (101), l'unité de calcul (200) étant par ailleurs conçue pour calculer le profil ionique du liquide en fonction de la vitesse d'écoulement (106) du liquide relevée.

12. Appareil ménager à circulation d'eau selon l'une des revendications 1 à 11, **caractérisé en ce que** le liquide est de l'eau.

13. Appareil ménager à circulation d'eau selon la revendication 12, **caractérisé en ce que** le profil ionique de l'eau comporte une concentration ionique pour des ions calcium (142) et une concentration ionique pour des ions de magnésium (144).

14. Appareil ménager à circulation d'eau selon l'une des revendications 1 à 13, **caractérisé en ce que** l'appareil ménager est prévu sous forme d'une machine à laver la vaisselle, une machine à laver, un four à vapeur, une machine à café, une bouilloire, un chauffe-eau, un réservoir d'eau chaude et/ou un adoucisseur d'eau.

15. Procédé d'opération d'un appareil ménager (1) à circulation d'eau comportant un dispositif capteur (100) pour déterminer un profil ionique d'un liquide, le profil ionique comprenant une information sur la concentration de différents types d'ions contenus dans le liquide, le dispositif capteur (100) comportant une section de conduite (101) permettant le passage du liquide, une première électrode (110), une seconde électrode (120) et un certain nombre d'éléments capteurs (130), la première électrode (110), la seconde électrode (120) et la section de conduite (101) étant disposées les unes par rapport aux autres de telle sorte que, suite à l'application d'une tension (U) entre la première électrode (110) et la seconde électrode (120), un champ électrique (E) se constitue dans au moins un tronçon (102) de la section de conduite (101), les éléments capteurs (130) étant disposés sur un bord de la section de conduite (101) et aptes à capter une propriété locale du liquide passant par la section de conduite (101), comprenant les étapes suivantes :
faire passer (S1) le liquide par la section de conduite (101), appliquer (S2) la tension (U) entre la première électrode (110) et la seconde électrode (120), capter (S3) la propriété locale du liquide à l'aide de chacun des éléments capteurs (130), et
déterminer (S4) le profil ionique du liquide en fonction de la propriété locale du liquide déterminée et du champ électrique (E).
